# EUROPEAN PATENT SPECIFICATION

(11) **EP 3 297 749 B1**
(45) Date of publication and mention of the grant of the patent: **13.03.2019**
(21) Application number: 16729199.6
(22) Date of filing: 19.05.2016
(51) Int. Cl.: B01D 61/18

(54) **MEMBRANE CONNECTOR TO PROMOTE MASS TRANSFER IN A MEMBRANE AERATED BIOFILM REACTOR (MABR)**
MEMBRANVERBINDER ZUR FÖRDERUNG DES STOFFTRANSPORTS IN EINEM BELÜFTETEN MEMBRAN-BIOFILMREAKTOR (MABR)
CONNECTEUR À MEMBRANE PERMETTANT DE FAVORISER LE TRANSFERT DE MASSE DANS UN RÉACTEUR À MEMBRANE À BIOFILM AÉRÉ (MABR)

(30) Priority: 19.05.2015 EP 15168280
(43) Date of publication of application: 28.03.2018
(73) Proprietor: OxyMem Limited, Athlone, Co. Westmeath (IE)
(72) Inventor: SYRON, Eoin, Bray Co. Wicklow (IE); SEMMENS, Michael, St. Paul, Minnesota 55105 (US); CASEY, Eoin, Clonskeagh Dublin 14 (IE); BYRNE, Wayne, Rathcoffey Co. Kildare (IE)
(74) Representative: Carmody, Mark
(86) International application number: PCT/EP2016/061352
(87) International publication number: WO 2016/184996

(56) References cited:
- WO-A1-2005/016498
- WO-A1-2013/085318
- WO-A2-2008/130885
- CN-A- 101 234 817
- CN-A- 102 826 652
- JP-A- H04 210 213
- JP-A- 2001 205 054
- US-A1- 2004 035 779
- US-A1- 2006 037 896
- US-A1- 2007 131 605

## Description

### Field of the Invention

The invention relates to membrane aerated biofilm reactors (MABRs). In particular, the invention relates to a device for use in a MABR, the device capable of increasing the mass transfer between the wastewater and the membrane attached biofilm.

### Background to the Invention

As the world's population continues to grow, and more and more people move to live in urban environments, there are increased demands being placed on the wastewater treatment infrastructure. Both municipal and industrial wastewater treatment plants are having to achieve higher and higher water quality levels to meet regulatory standards, which at the same time must be achieved in smaller spaces, utilising less resources. This is driving innovation in wastewater treatment technology. One such technology which can achieve these increased treatment rates while at the same time utilising less resources is the Membrane Aerated Biofilm Reactor (MABR). The MABR utilises gas permeable membranes to provide oxygen to the pollutant degrading bacteria which grow attached to the membrane surface. The MABR combines the low energy requirements of permeable membrane aeration with the key advantages of biofilm-based processes such as the high volumetric reaction rate that can be attained due the high specific biomass concentration. Biofilm based treatment systems have become more common in recent years. These systems generally place moving media into reactor tanks, the biofilm attaches to the media and both move about the tank freely with the liquid as it is mixed. Such Biofilm Reactors are described in US Patent No. 7189323.

Two issues remain with biofilm-based systems: 1) a lot of energy is required to provide both the oxygen necessary for the biofilm and the energy required to mix the water and the media while in these systems; 2) the depth to which the biofilm is active is very thin due the dissolved oxygen being consumed in the outer layers of the biofilm and results in the inner layers of the biofilm not being aerobically active. Both of these issues are overcome by the MABR.

The MABR has several advantages over conventional biofilm technologies:
(1) comparatively high volumetric carbon oxygen demand (COD) removal rates are achievable especially if pure oxygen is fully exploited and if biofilm thickness-control measures are in place.
(2) bubbleless aeration offers the potential for significantly higher oxygen utilization efficiencies with consequent energy savings. In addition, reduced air stripping during the biotreatment of volatile organic compounds is possible.
(3) simultaneous nitrification, denitrification and COD removal can be achieved at comparatively higher rates due to the unique microbial population stratification.
(4) specialist degrading microorganisms, such as ammonia oxidizing bacteria, tend to be preferentially grow adjacent to the biofilm-membrane interface thereby being protected from biofilm erosion.

The MABR technology is beginning to make the transition from research scale to full scale deployments. The problem being experienced by upscaling the size of the MABRs is that contact (mixing) between the pollutant-containing wastewater and the biofilm attached to the support media is inadequate. Therefore additional mixing and enhanced mass transfer is essential but this must be performed without a significant increase in energy as this would erode the major commercial advantage of the MABR.

WO2008130885 discloses a membrane fibre bunch for a MABR having a connector at each end with a circular profile which constrains the fibre bunch into a circular profile along its length. The disadvantage with this arrangement is that all the fibers are bunched together where the membrane enters the connector and not arranged into a specific distributed order. While the fibers maybe free to move they will experience the same force and due to excess length all fibres will move together The fibres will remain tightly bound together at the inlet of the connectors. JP 04210213 A and US 2004/0035779 disclose hollow fibre membrane bundle with elliptical or star-shaped cross-section, however in circular pottings.WO 2013/085318 dicloses zig-zag shaped hollow fibre bundles and pottings. US 2007/0131605 discloses rectangular bundles and pottings with a circular connector. CN 102826652 disclose race-track shaped pottings and bundles. In JP 2001205054 makes use of an elongated pottings and a funnel shaped connector.

It is an objective of the present invention to overcome at least one of the above-mentioned problems.

### Summary of the Invention

In the MABR, the biofilm is naturally immobilized on an oxygen permeable membrane. Oxygen diffuses through the membrane into the biofilm where oxidation of pollutants, supplied at the biofilm-liquid interface, takes place. The oxygen supply rate is controlled by the intra-membrane oxygen partial pressure (a process parameter) and membrane surface area (a design parameter). The object of the present application is to maximise the mass transfer of oxygen from the gas within the membranes of the MABR to the biofilm on the membrane surface. It is also an object of the present invention to maximise the rate of mass transfer of the pollutants from the wastewater into the pollutant degrading biofilm by exposing more of membrane fibre surface to the polluted wastewater. These combined advantages of the invention increase the rate of wastewater treatment of the MABR. The arrangement of the membrane fibres into groups with unique and innovative cross sectional shapes avoids, prevents, and/or minimises the occurrence of dead zones or nonreactive zones or stagnant zones within the membrane bunch and in between the membrane bunches when they are arranged into cassettes and modules within the reactor. The inclusion of the unique membrane arrangement not only increases the exposed surface area of the membranes but, due to the non-uniform arrangement of these membrane groups, turbulence is created in the liquid flow, thus further improving the mass transfer from the membranes and to the biofilm.

To ensure the MABR can become an effective full-scale technology for wastewater treatment, there is a critical need to ensure that the reactor is well mixed and that there is effective contact between as much of the surface area of the membrane-attached pollutant-degrading biofilm and the polluted wastewater to be treated. The Applicants have provided a solution for maximising the contact between the wastewater and the pollutant degrading biofilm in a MABR treatment system or tank. The solution creates increased mixing due to turbulence within the system and without any additional energy requirement. The system thereby maintains the low energy demand of the MABR for biological wastewater treatment.

This is done in a way that is easy to manufacture and assemble, and does not require the addition of a weave or wrap.

According to the present invention, there is provided, as set out in the appended claims, a membrane fibre according to cl.1.

In one embodiment, the bunch of membrane fibres are bound together by a semi-rigid or rigid connector. Preferably, the connector or overmold has a preformed profile shape.

The connector or overmold has a non-circular profile as defined in claim 1. This has been found to increase mass transfer of gas from the fibres in the bunch due to exposure of more surface area of the fibres to the surrounding liquid. Importantly, the noncircular profile of the fibre bunches has been found to cause an increase in turbulence of the liquid surrounding the bunch, thereby improving localised mixing and reducing the thickness of the boundry layer formed on the fibres.

In one embodiment, the profile of the connector or overmold is elongated. The profile may be elongated along one radial axis (i.e. ellipsoidal - Fig. 1) or along different radial axes (i.e. a three, four, or five pointed star - Figs 2 and 3).

The connector or overmold is shaped to funnel the membrane fibres towards the manifold. This means that the connector or overmold forces the bunch of fibres to taper inwardly towards the manifold.

The connector or overmold has an inlet section having a non-circular profile, an outlet configured for connection to a manifold having a circular profile, and a funnel section intermediate the inlet and outlet sections. The profile of the funnel section transitions from the non-circular profile at an inlet end to a circular profile at an outlet end.

The connector and overmold are neither circular nor cylindrical in profile. A unique profile may be created by means of placing the bunch of membrane fibres into (i) a pre-formed connector or (ii) by means of holding the bunch of membrane fibres in the unique profile and fixing them in this configuration by means of placing an adhesive over the fibers so as to maintain the fibres in this configuration or by moulding the connector around the fibres to keep them in place (an overmold). The connector or overmold has a profile shape selected from the group comprising an ellipse, a three-pointed star, a four-pointed star, a five-pointed star, a cross-shape, to provide a more widely distributed and mixed flow across the entire bunch of membrane fibres.
In one embodiment, the bunch of membrane fibres are attached at either end to an air supply manifold.
In one embodiment, the group of membrane fibres of the bunch extend into the housing and away from a central axis of the group of membrane fibres, and maximises an exposed surface area of the membrane fibres.

In one embodiment, the connector is configured to be attached to an air supply manifold. Preferably, the connector is attached to the air supply manifold by a push-fit type fitting or affixed directly thereto.

In one embodiment there is provided a cassette comprising a plurality of membrane fibre bunches described above, or a plurality of arrays described above, arranged side-by-side and attached to an upper and lower air manifold.

In one embodiment, there is provided a membrane-aerated biofilm reactor (MABR) of the type comprising a housing and at least one membrane fibre bunch as described above.

In one embodiment, there is provided a membrane-aerated biofilm reactor (MABR) comprising a housing and at least one array as described above.

In one embodiment, there is provided a membrane-aerated biofilm reactor (MABR) comprising a housing and at least one cassette described above.

In one embodiment, a plurality of membrane fibre bunches are placed in sequence to form an array. Ideally, each membrane fibre bunch in the array share a common air supply manifold. Each membrane fibre bunch are in fluid communication with each other.

In one embodiment, the array comprises a plurality of membrane fibre bunches placed side by side, which forms a cassette. Preferably, several cassettes are placed together to form a module, which when placed within the housing, substantially occupies the space within the housing of the MABR. Ideally, each membrane fibre bunch in the array is attached to an upper and lower air manifold to form the cassette. The cassette is then placed into the housing and fixed in position to form a MABR. The positioning of the membrane fibre bunches in the array provides a well distributed and mixed flow throughout all of the membrane fibre bunches in the array (also known as membrane cassettes).

In one embodiment, the profile of the group of membrane fibres in the membrane bunch may be narrow and extended. The narrowing and extending of the profile of the membrane fibres in the membrane bunch reduces the distance between (all of) the fibres and any bulk liquid to be treated. This permits a more widely distributed and mixed flow across the entire bunch of membrane fibres.

In one embodiment, the connector is configured to be attached to an air supply manifold. The connector can be attached to the air supply manifold via a fitting, or adhered directly to the manifold with any suitable adhesive known to the skilled person such as glue, epoxy resin and the like.

The extension of the membrane fibres into the liquid to be treated, and away from the central axis of the bunch of vertical membrane fibres, reduces the distance between the central membrane fibres and the liquid, and maximises the exposed surface area of the membrane fibres. The arrangement of membrane fibres in the bunch provides a narrow profile, reducing the distance between all of the membrane fibres and any liquid in the housing to be treated. The configuration of the membrane fibres in the bunch provides a higher effective rate of oxygen transfer in the reactor.

In the specification, the term "Membrane Aerated Biofilm Reactor (MABR)" should be understood to mean a membrane supported biofilm reactor (MSBR) or Membrane Biofilm Reactor (MBfR) for treating water and or wastewater liquids to remove carbonaceous pollutant removal, nitrify/denitrify the pollutants, and/or perform xenobiotic biotreatment of the wastewater constituents, and employing an air/oxygen/hydrogen (gas) permeable membrane (often a hollow fibre membrane) that provides an interface between the fluid to be treated (fluid phase) and an air/oxygen/hydrogen supply (gas phase). Soluble organic compounds in the liquid are supplied to the biofilm from the biofilm-liquid interface, whereas air/oxygen/hydrogen/carbon dioxide or other gas supply to the biofilm is from the biofilm-membrane interface (by diffusing through the membrane). Typically, a biofilm consisting of a heterogeneous population of bacteria (micro-organisims) (generally including nitrifying, denitrifying, and heterotrophic, bacteria) grows on the fluid phase side of the membrane. MABRs can achieve bubble-less aeration and high oxygen utilization efficiency (up to 100%) and the biofilm can be separated into aerobic/anoxic/anaerobic zones to simultaneously achieve removal of carbonaceous organic pollutants, as well as nitrification and denitrification in a single biofilm. An example of MABRs of the type comprising a lumen containing a gas phase, a liquid phase, and a gas permeable membrane providing an interface between the gas and liquid phases are described by European Patent No. 2 361 367 (University College Dublin).
In the specification, the term "bunch of membrane fibres" should be understood to mean a plurality of membrane fibres arranged together as a bunch as distinct from a planar arrangement of fibres such as a sheet. The number of membrane fibres in a single bunch can be any number, for example from 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 membrane fibres. Typically, the fibres in a bunch are parallel and ideally co-extensive.
In the specification, the term "membrane cassette" should be understood to mean a plurality of bunches of membrane fibres arranged side-by-side and typically attached to an upper and lower air manifold. The number of bunches of membrane fibres in a cassette can be any number, for example from 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 bunches of membrane fibres.

In the specification, the term "housing" should be understood to mean a holding vessel, such as a tank, within which is placed in the MABR. The term "housing" and "tank" may be used interchangeably. The term includes closed casings as well as open tanks.

In the specification, the terms "upper manifold" and "lower manifold" should be understood to mean air manifolds connected to either ends of the hollow fibre membranes. The manifolds provide a means to supply and remove the gas to/from the lumen of the membranes. If the fibres are placed in the vertical orientation, the manifolds are then in the upper and lower portions of the MABR housing.

In the specification, the term "semi-rigid", in the context of the connector, should be understood to mean partly or moderately rigid, having a slight tendency to be pliable under air or applied pressure, and naturally returning back to an original state while not compromising the integrity of the connector structure.

### Brief Description of the Drawings

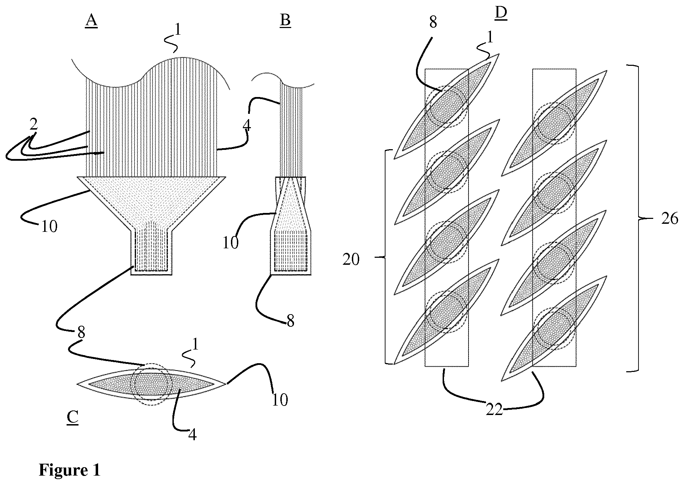

The invention will be more clearly understood from the following description of an embodiment thereof, given by way of example only, with reference to the accompanying drawings, in which:-
**Figure 1** illustrates (A) an elevation view, (B) a side view and (C) a plan view of one embodiment of a bunch of membrane fibres arranged by a semi-rigid/rigid connector or overmold to form an array with a particular profile for use in a membrane aerated biofilm reactor (MABR) device. Figure 1(D) illustrates a plan view of two adjoining arrays of the claimed invention. The bunches of membrane fibres are arranged into a single array, which can be placed side-by-side with other arrays to form a cassette and distributed flow.

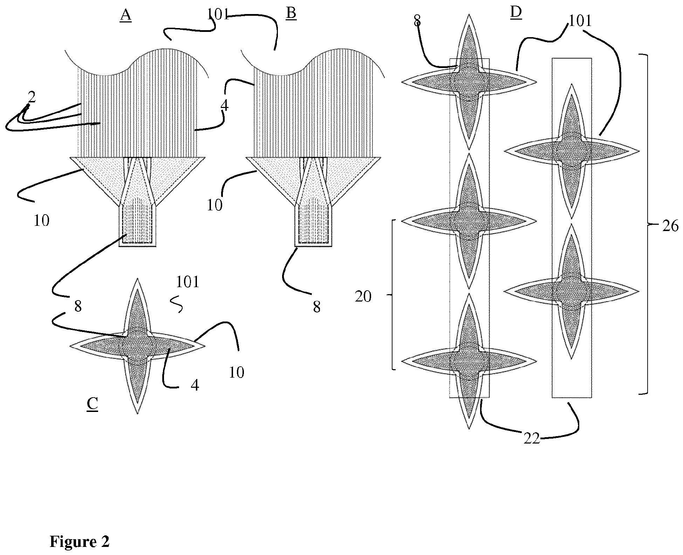

**Figure 2** illustrates (A) an elevation view, (B) a side view and (C) a plan view of one embodiment of a bunch of fibres arranged by a semi-rigid/rigid connector or overmold to form an array with a particular profile for use in the membrane aerated biofilm reactor (MABR) device. Figure 2(D) illustrates a plan view two adjoining arrays of the claimed invention. The bunches of membrane fibres are arranged into a single array, which can be placed side-by-side with other arrays to form a cassette and distributed flow.

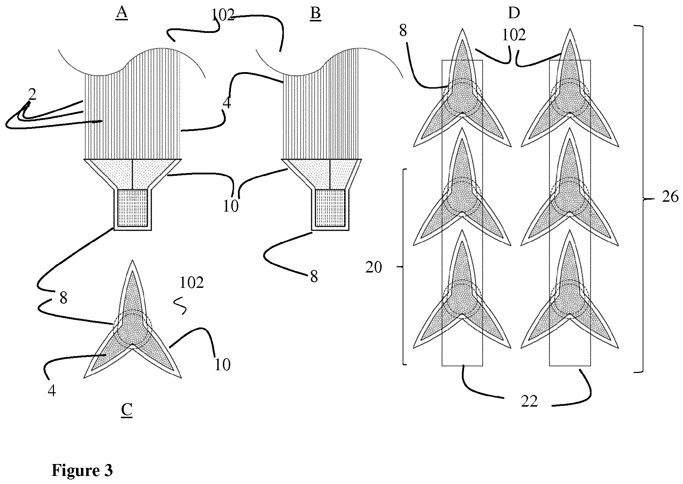

**Figure 3** illustrates (A) an elevation view, (B) a side view and (C) a plan view of one embodiment of a bunch of fibres arranged by a semi-rigid/rigid connector or overmold to form an array with a particular profile for use in the membrane aerated biofilm reactor (MABR) device. Figure 3(D) illustrates a plan view two adjoining arrays of the claimed invention. The bunches of membrane fibres are arranged into a single array, which can be placed side-by-side with other arrays to form a cassette and distributed flow.

### Detailed Description of the Drawings

The invention relates to a device for treating wastewater liquids, the device is suitable for use a membrane supported biofilm reactor (MSBR) or a Membrane Aerated Biofilm Reactor (MABR). The device comprising a plurality of membranes arranged into a bunch having a specific profile/configuration, with each membrane consisting of a lumen containing a gas phase, a liquid phase, and a gas permeable membrane providing an interface between the gas and liquid phases; and a means for holding the membranes in a defined configuration to maintain a high exposure of the membrane's exposed surface area and ensure that the membranes are adequately dispersed in the wastewater

The membrane bunches which are held in the defined configuration are then placed in an array with a gas manifold at either end of the membrane bunches allowing for air/oxygen/hydrogen/methane/carbon dioxide or other gas to be supplied to the membrane lumen and removed from the membrane lumen at the opposite side. The array of bunches along with the manifold (cassette) are then arranged in the housing to maximise the contact between the membranes and the liquid contained in the housing.

Referring now to the figures, where **Figure 1** illustrates a general embodiment of the device of the present invention. Specifically, **Figures 1A** to **1C** illustrate (A) an elevation view, (B) a side view and (C) a plan view of a typical device of the present invention, and is generally referred to by reference numeral 1. The device 1 comprises a group of membrane fibres 2 arranged together as a bunch of vertical membrane fibres 4 attached at either end to an air supply manifold 8. A biofilm is grown and accumulates upon the densely packed bunch of membrane fibres 4. Each membrane fibre 2 has a lumen containing a gas phase. The bunch of membrane fibres 4 are connected and arranged together by a connector or overmold 10. The connector or overmold 10 is configured to arrange and maintain the bunch of membrane fibres 4 in a spaced-apart configuration from one another. This provides the device 1 with a group of membrane fibres 2 that allows the gas to flow within the lumen of each of the membranes 2. The connector or overmold 10 also imparts a unique profile on the bunch of membranes 4, such as the elliptical profile illustrated in Figure 1. As illustrated in **Figure 1** (and **Figures 2** **and** **3**), the connector or overmold 10 is configured to attach to the air supply manifold 8.

When a plurality of a bunch of membrane fibres 4 are arranged by a semi-rigid/rigid connector or overmold 10 and placed in sequence, an array 20 is formed. The array 20 now comprises a plurality of membrane fibre bunches 4 with a particular profile imparted to them by the connector or overmold 10. Each bunch of membrane fibres 4 in the array 20 are attached to an upper and lower air manifold 22 to form a cassette 26. Figure 1(D) illustrates two adjoining cassettes 26 comprising the arrays 20 of the claimed invention.

The positioning of the membrane fibre bunches 4 in the array 20 provides a well distributed and mixed flow throughout all of the membrane fibre bunches 4 in the array 20. The cassette 26 can be placed and fixed within a housing of a membrane aerated biofilm reactor (MABR) device to form a module.

Referring to **Figures 2** and **3**, there is illustrated additional embodiments of the device in which parts or steps described with reference to the previous embodiments are assigned the same numerals. In the embodiment, the device 200, 300 comprise a group of membrane fibres 2 arranged together as a bunch of vertical membrane fibres 4 attached at either end to an air supply manifold 8. A biofilm is grown and accumulates upon the densely packed bunch of membrane fibres 4. Each membrane fibre 2 has a lumen containing a gas phase. The bunch of membrane fibres 4 are connected and arranged together by a connector or overmold 101, 102. The connector or overmold 101, 102 is configured to arrange and maintain the bunch of membrane fibres 4 in a spaced-apart configuration from one another. This provides the device 200, 300 with a group of membrane fibres 2 that allows the gas to flow within the lumen of each of the membranes 2. The connector or overmold 101, 102 also imparts a unique profile on the bunch of membranes 4, such as the four-pointed star profile illustrated in Figure 2 and the three-pointed star profile illustrated in Figure 3. It should be understood that the profile of the device 1, 101, 102 can be any profile that provides a more widely distributed and mixed flow across the entire bunch of membrane fibres 4, such as a five-pointed star, a cross-shape, a chevron, and the like.

From a biocatalytic point of view, the more membranes and biofilm in a wastewater treatment housing the better. However, above a certain limit, the accumulation of biofilm can cause severe problems with liquid flow distribution. Therefore, an effective membrane distribution must be maintained. Commercially, the membrane packing density must be increased to provide the most gas transferring membranes per unit of reactor volume. Many of the laboratory scale studies reported to-date in the literature were operated with low membrane packing densities. This current invention describes a device 1, 101, 102 to allow for better distribution of high packing densities of membrane aeration fibres within a membrane aerated biofilm reactor. The current invention also results in effective contact of the liquid waste with all of the membranes 2 in the reactor.

## Claims

1. A membrane fibre bunch for use in a membrane-aerated biofilm reactor (MABR), having a housing, the membrane fibre bunch comprising: a group of membrane fibres (2) arranged in a bunch (4), with each fibre having a lumen containing a gas phase; and a means for connecting the group of membrane fibres to a manifold so that in use gas can flow within the lumen of the membrane; wherein the membrane fibres in the bunch are arranged together by a connector or overmold (10), the connector or overmold configured to arrange and maintain the membrane fibres in the bunch in a spaced-apart configuration from one another, the connector or overmold for receiving the membrane fibres having an inlet with a non-circular cross-sectional shape, an outlet configured for connection to a manifold (8) having a circular shape and a funnel section intermediate the inlet and outlet sections, wherein the inlet of the connector or overmold and membrane fibre bunch have a non-circular cross-sectional elongated shape along at least one radial axis or along at least two different radial axes; in which the connector or overmold funnels the membrane fibres towards the manifold, **characterized in that** the non-circular profile shape of the inlet of the connector or overmold is selected from the group consisting of an ellipse, a three-pointed star, a four-pointed star, a five-pointed star, or a cross-shape.

2. A membrane fibre bunch according to Claim 1, wherein the membrane fibre bunch is attached at either end to an air supply manifold and a connector or overmold.

3. A membrane fibre bunch according to Claim 1 or Claim 2, wherein the or each connector or overmold is configured to be attached separately to an air supply manifold.

4. A membrane fibre bunch according to Claim 3, wherein the or each connector or manifold is attached to the air supply manifold by a push-fit type fitting or affixed directly thereto.

5. A membrane fibre bunch according to any one of the preceding claims, wherein a number of membrane fibre bunches arranged side-by-side, and attached to the air supply manifold, are disposed within the housing to form a cassette.

6. A membrane fibre bunch according to Claim 5, in which each bunch is separated from another bunch by a separate overmold.

7. An array comprising a plurality of membrane fibre bunches of any of Claims 1 to 6 arranged in sequence; and in which the plurality of membrane fibre bunches are optionally in fluid communication with each other; and in which each membrane fibre bunch optionally comprises a separate connector or overmold.

8. A membrane cassette comprising a plurality of arrays of membrane fibre bunches of Claim 7 arranged side-by-side and attached to an upper and lower air manifold.

9. A membrane-aerated biofilm reactor (MABR) of the type comprising a housing and at least one membrane fibre bunch as claimed in any of Claims 1 to 6.

10. A membrane-aerated biofilm reactor (MABR) comprising a housing and at least one array according to Claim 7.

11. A membrane-aerated biofilm reactor (MABR) comprising a housing and at least one cassette according to Claim 8.

12. A membrane fibre bunch assembly for use in a membrane-aerated biofilm reactor (MABR) comprising the membrane fibre bunch as claimed in any one of Claims 1 to 7 with the connector or overmold disposed at each end of the membrane fibre bunch and configured to constrain the bunch of fibres in a non-circular cross-sectional shape along a substantial length of the fibres.

13. A membrane fibre bunch assembly as claimed in Claim 12 including a plurality of groups of membrane fibres arranged in a bunch, in which each membrane fibre bunch comprises a connector or overmold disposed at each end of the membrane fibre bunch.

14. A membrane cassette for use in a membrane-aerated biofilm reactor and comprising at least one membrane fibre bunch according to any of Claims 1 to 6 and a gas inlet manifold and a gas outlet manifold operatively connected to the connector or overmold disposed at each end of the at least one membrane fibre bunch.

15. A membrane-aerated biofilm reactor comprising a membrane fibre bunch of any of Claims 1 to 6 or a membrane cassette according to Claim 14.

## Patentansprüche

1. Membranfaserbündel zur Verwendung in einem membranbelüfteten Biofilmreaktor (MABR) mit einem Gehäuse, wobei das Membranfaserbündel Folgendes umfasst: eine Gruppe von in einem Bündel (4) angeordneten Membranfasern (2), wobei jede Faser ein eine Gasphase enthaltendes Lumen aufweist; und ein Mittel zum Verbinden der Gruppe von Membranfasern mit einem Verteiler, sodass in Gebrauch Gas in dem Lumen der Membran strömen kann; wobei die Membranfasern in dem Bündel durch einen Verbinder oder eine Umspritzung (10) zusammen angeordnet sind, der Verbinder oder die Umspritzung dazu konfiguriert ist, die Membranfasern in dem Bündel in einer voneinander beabstandeten Konfiguration anzuordnen und zu halten, der Verbinder oder die Umspritzung zum Aufnehmen der Membranfasern einen Einlass mit einer nicht-kreisförmigen Querschnittsform, einen zur Verbindung mit einem Verteiler (8) konfigurierten Auslass mit einer Kreisform und einen Trichterabschnitt zwischen dem Einlass- und dem Auslassabschnitt aufweist, wobei der Einlass des Verbinders oder der Umspritzung und das Membranfaserbündel entlang mindestens einer radialen Achse oder entlang mindestens zwei verschiedener radialer Achsen eine nicht-kreisförmige langgestreckte Querschnittsform aufweisen; wobei der Verbinder oder die Umspritzung die Membranfasern in Richtung des Verteilers trichterförmig zusammenführt, **dadurch gekennzeichnet, dass** die nicht-kreisförmige Profilform des Einlasses des Verbinders oder der Umspritzung aus der Gruppe ausgewählt ist, die aus einer Ellipse, einem dreizackigen Stern, einem vierzackigen Stern, einem fünfzackigen Stern oder einer Kreuzform besteht.

2. Membranfaserbündel nach Anspruch 1, wobei das Membranfaserbündel an beiden Enden an einem Luftzufuhrverteiler und einem Verbinder oder einer Umspritzung angebracht ist.

3. Membranfaserbündel nach Anspruch 1 oder Anspruch 2, wobei der oder jeder Verbinder oder die oder jede Umspritzung dazu konfiguriert ist, separat an einem Luftzufuhrverteiler angebracht zu werden.

4. Membranfaserbündel nach Anspruch 3, wobei der oder jeder Verbinder oder Verteiler mit einer Steckverbindung an dem Luftzufuhrverteiler angebracht oder direkt daran befestigt ist.

5. Membranfaserbündel nach einem der vorangehenden Ansprüche, wobei eine Anzahl von nebeneinander angeordneten und an dem Luftzufuhrverteiler angebrachten Membranfaserbündeln in dem Gehäuse angeordnet sind, um eine Kassette zu bilden.

6. Membranfaserbündel nach Anspruch 5, wobei jedes Bündel durch eine separate Umspritzung von einem anderen Bündel getrennt ist.

7. Anordnung, umfassend eine Vielzahl von Membranfaserbündeln nach einem der Ansprüche 1 bis 6, die der Reihe nach angeordnet sind; und wobei sich die Vielzahl von Membranfaserbündeln optional in Fluidverbindung miteinander befinden; und wobei jedes Membranfaserbündel optional einen separaten Verbinder oder eine separate Umspritzung umfasst.

8. Membrankassette, umfassend eine Vielzahl von Anordnungen von Membranfaserbündeln nach Anspruch 7, die nebeneinander angeordnet und an einem unteren und einem oberen Luftverteiler angebracht sind.

9. Membranbelüfteter Biofilmreaktor (MABR) der Art, die ein Gehäuse und mindestens ein Membranfaserbündel nach einem der Ansprüche 1 bis 6 umfasst.

10. Membranbelüfteter Biofilmreaktor (MABR), umfassend ein Gehäuse und mindestens eine Anordnung nach Anspruch 7.

11. Membranbelüfteter Biofilmreaktor (MABR), umfassend ein Gehäuse und mindestens eine Kassette nach Anspruch 8.

12. Membranfaserbündelbaugruppe zur Verwendung in einem membranbelüfteten Biofilmreaktor (MABR), umfassend das Membranfaserbündel nach einem der Ansprüche 1 bis 7, wobei der Verbinder oder die Umspritzung an jedem Ende des Membranfaserbündels angeordnet ist und dazu konfiguriert ist, das Bündel von Fasern entlang einer wesentlichen Länge der Fasern ein eine nicht-kreisförmige Querschnittsform zu zwängen.

13. Membranfaserbündelbaugruppe nach Anspruch 12, umfassend eine Vielzahl von Gruppen von in einem Bündel angeordneten Membranfasern, wobei jedes Membranfaserbündel an jedem Ende des Membranfaserbündels angeordnet einen Verbinder oder eine Umspritzung umfasst.

14. Membrankassette zur Verwendung in einem membranbelüfteten Biofilmreaktor, und umfassend mindestens ein Membranfaserbündel nach einem der Ansprüche 1 bis 6 und einen Gaseinlassverteiler und einen Gasauslassverteiler, die wirksam mit dem bzw. der an jedem Ende des mindestens einen Membranfaserbündels angeordneten Verbinder oder Umspritzung verbunden sind.

15. Membranbelüfteter Biofilmreaktor, umfassend ein Membranfaserbündel nach einem der Ansprüche 1 bis 6 oder eine Membrankassette nach Anspruch 14.

## Revendications

1. Faisceau de fibres membranaires pour une utilisation dans un réacteur à biofilm aéré sur membrane (MABR) ayant un boîtier, le faisceau de fibres membranaires comprenant : un groupe de fibres membranaires (2) agencées dans un faisceau (4), chaque fibre ayant une lumière contenant une phase gazeuse ; et un moyen destiné à connecter le groupe de fibres membranaires à un collecteur de sorte que, lors de l'utilisation, du gaz peut s'écouler à l'intérieur de la lumière de la membrane ; où les fibres membranaires dans le faisceau sont agencées ensemble par un connecteur ou surmoulage (10), le connecteur ou surmoulage étant configuré pour agencer et maintenir les fibres membranaires dans le faisceau dans une configuration espacée les unes des autres, le connecteur ou surmoulage destiné à recevoir les fibres membranaires ayant une entrée avec une forme de section transversale non circulaire, une sortie configurée pour une connexion à un collecteur (8) ayant une forme circulaire et une section en entonnoir entre les sections d'entrée et de sortie, où l'entrée du connecteur ou surmoulage et le faisceau de fibres membranaires ont une forme allongée de section transversale non circulaire le long d'au moins un axe radial ou le long d'au moins deux axes radiaux différents ; dans lequel le connecteur ou surmoulage achemine les fibres membranaires vers le collecteur, **caractérisé en ce que** la forme de profil non circulaire de l'entrée du connecteur ou surmoulage est choisie dans le groupe constitué par une ellipse, une étoile à trois branches, une étoile à quatre branches, une étoile à cinq branches, ou une forme de croix.

2. Faisceau de fibres membranaires selon la revendication 1, le faisceau de fibres membranaires étant attaché à chaque extrémité à un collecteur d'alimentation en air et un connecteur ou surmoulage.

3. Faisceau de fibres membranaires selon la revendication 1 ou la revendication 2, dans lequel le ou chaque connecteur ou surmoulage est configuré pour être attaché séparément à un collecteur d'alimentation en air.

4. Faisceau de fibres membranaires selon la revendication 3, dans lequel le ou chaque connecteur ou collecteur est attaché au collecteur d'alimentation en air par un raccord de type enfichable par pression ou fixé directement sur celui-ci.

5. Faisceau de fibres membranaires selon l'une quelconque des revendications précédentes, dans lequel un certain nombre de faisceaux de fibres membranaires agencés côte à côte, et attachés au collecteur d'alimentation en air, sont disposés à l'intérieur du boîtier pour former une cassette.

6. Faisceau de fibres membranaires selon la revendication 5, dans lequel chaque faisceau est séparé d'un autre faisceau par un surmoulage séparé.

7. Réseau comprenant une pluralité de faisceaux de fibres membranaires selon l'une quelconque des revendications 1 à 6 agencés en séquence ; et dans lequel les faisceaux de la pluralité de faisceaux de fibres membranaires sont facultativement en communication fluidique les uns avec les autres ; et dans lequel chaque faisceau de fibres membranaires comprend facultativement un connecteur ou surmoulage séparé.

8. Cassette membranaire comprenant une pluralité de réseaux de faisceaux de fibres membranaires selon la revendication 7 agencés côte à côte et attachés à un collecteur d'air supérieur et inférieur.

9. Réacteur à biofilm aéré sur membrane (MABR) du type comprenant un boîtier et au moins un faisceau de fibres membranaires tel que revendiqué dans l'une quelconque des revendications 1 à 6.

10. Réacteur à biofilm aéré sur membrane (MABR) comprenant un boîtier et au moins un réseau selon la revendication 7.

11. Réacteur à biofilm aéré sur membrane (MABR) comprenant un boîtier et au moins une cassette selon la revendication 8.

12. Ensemble de faisceau de fibres membranaires pour une utilisation dans un réacteur à biofilm aéré sur membrane (MABR) comprenant le faisceau de fibres membranaires tel que revendiqué dans l'une quelconque des revendications 1 à 7 avec le connecteur ou surmoulage disposé à chaque extrémité du faisceau de fibres membranaires et configuré pour contraindre le faisceau de fibres dans une forme de section transversale non circulaire le long d'une longueur substantielle des fibres.

13. Ensemble de faisceau de fibres membranaires tel que revendiqué dans la revendication 12 incluant une pluralité de groupes de fibres membranaires agencés en un faisceau, dans lequel chaque faisceau de fibres membranaires comprend un connecteur ou surmoulage disposé à chaque extrémité du faisceau de fibres membranaires.

14. Cassette membranaire pour une utilisation dans un réacteur à biofilm aéré sur membrane et comprenant au moins un faisceau de fibres membranaires selon l'une quelconque des revendications 1 à 6 et un collecteur d'entrée de gaz et un collecteur de sortie de gaz connectés de manière fonctionnelle au connecteur ou surmoulage disposé à chaque extrémité de l'au moins un faisceau de fibres membranaires.

15. Réacteur à biofilm aéré sur membrane comprenant un faisceau de fibres membranaires selon l'une quelconque des revendications 1 à 6 ou une cassette membranaire selon la revendication 14.
